(19) Europäisches Patentamt
European Patent Office
Office européen des brevets

(11) EP 0 992 545 B1

(12) **EUROPÄISCHE PATENTSCHRIFT**

(45) Veröffentlichungstag und Bekanntmachung des Hinweises auf die Patenterteilung:
**11.12.2002 Patentblatt 2002/50**

(51) Int Cl.[7]: **C09B 43/16**, C09B 43/145, C09D 11/02

(21) Anmeldenummer: **99118057.1**

(22) Anmeldetag: **22.09.1999**

(54) **Azofarbstoffe und diese enthaltende Drucktinten**

Azo dyes and printing inks containing them

Colorants azoiques et encres d'impression les contenant

(84) Benannte Vertragsstaaten:
**CH DE FR GB IT LI**

(30) Priorität: **05.10.1998 DE 19845640**

(43) Veröffentlichungstag der Anmeldung:
**12.04.2000 Patentblatt 2000/15**

(73) Patentinhaber: **Bayer Aktiengesellschaft**
**51368 Leverkusen (DE)**

(72) Erfinder:
- **Kunde, Klaus, Dr.**
  **53819 Neunkirchen-Seelscheid (DE)**
- **Jörss, Michael-Thomas**
  **50169 Kerpen (DE)**
- **Wild, Peter, Dr.**
  **51519 Odenthal (DE)**

(56) Entgegenhaltungen:
**EP-A- 0 024 668**
**EP-A- 0 065 479**
**EP-A- 0 755 984**
**EP-A- 0 794 233**
**WO-A-96/24636**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 992 545 B1

Printed by Jouve, 75001 PARIS (FR)

## Beschreibung

Die Erfindung betrifft neue Azofarbstoffe, ein Verfahren zur ihrer Herstellung sowie ihre Verwendung zum Färben und Bedrucken von Papier sowie anderen Materialien, insbesondere als Farbstoff in Ink Jet-Tinten.

Die erfindungsgemäßen Farbstoffe besitzen in Form ihrer freien Säuren die Formel (I)

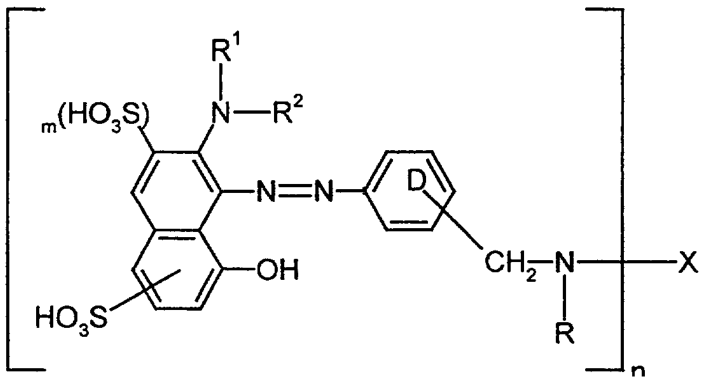

(I),

worin

| | |
|---|---|
| R | für Wasserstoff oder gegebenenfalls substituiertes $C_1$-$C_6$-Alkyl steht, |
| $R^1$ | Wasserstoff oder $C_1$-$C_6$-Alkyl bedeutet, |
| $R^2$ | Wasserstoff, gegebenenfalls substituiertes $C_1$-$C_6$-Alkyl oder gegebenenfalls substituiertes Phenyl bedeutet, |
| der Ring D | gegebenenfalls durch ein oder mehrere gleiche oder verschiedene Substituenten aus der Reihe $C_1$-$C_6$-Alkyl, $C_1$-$C_6$-Alkoxy, Halogen, insbesondere F, Cl und Br und $SO_3H$ substituiert ist, |
| m | 0 oder 1 bedeutet, |
| n | 1, 2 oder 3 bedeutet und in Abhängigkeit von n |
| X | für einen mono-, di- oder trivalenten Acylrest, Pyrimidin- oder 1,3,5-Triazinrest steht, der nicht faserreaktiv ist. |

Als beispielhafte Substituenten für gegebenenfalls substituierte Alkylreste kommen beispielsweise in Frage: OH, $NR^3R^4$, wobei $R^3$ und $R^4$ unabhängig voneinander für Wasserstoff, $C_1$-$C_4$-Alkyl, $C_2$-$C_4$-Hydroxyalkyl stehen.

Als beispielhafte Substituenten für gegebenenfalls substituiertes Phenyl kommen beispielsweise in Frage: $C_1$-$C_6$-Alkyl, $C_1$-$C_6$-Alkoxy, COOH, $SO_3H$, F, Cl, Br.

Bevorzugte Farbstoffe der Formel (I) sind solche, worin

| | |
|---|---|
| R | für Wasserstoff, $C_1$-$C_6$-Alkyl oder $C_2$-$C_4$-Hydroxyalkyl steht, |
| $R^1$ | Wasserstoff oder $C_1$-$C_6$-Alkyl bedeutet, |
| $R^2$ | Wasserstoff, $C_1$-$C_6$-Alkyl, $C_2$-$C_4$-Hydroxyalkyl, $C_2$-$C_4$-Aminoalkyl, insbesondere durch $NR^3R^4$ substituiertes $C_2$-$C_4$-Alkyl bedeutet, worin |
| $R^3$ und $R^4$ | unabhängig voneinander für Wasserstoff, $C_1$-$C_4$-Alkyl und $C_2$-$C_4$-Hydroxyalkyl stehen, oder gegebenenfalls durch $C_1$-$C_4$-Alkyl, $C_1$-$C_4$-Alkoxy, COOH, $SO_3H$, F, Cl und/oder Br substituiertes Phenyl bedeuten und |
| der Ring D | gegebenenfalls substituiert ist durch einen oder mehrere gleiche oder verschiedene Substituenten aus der Reihe $C_1$-$C_4$-Alkyl, $C_1$-$C_4$-Alkoxy, F, Cl, Br und $SO_3H$. |

In einer besonderen Ausführungsform bedeutet

X im Falle n = 1 gegebenenfalls durch COOH substituiertes $C_2$-$C_6$-Alkanoyl, gegebenenfalls durch $C_1$-$C_4$-Alkyl, $C_1$-$C_4$-Alkoxy, $SO_3H$, COOH, F, Cl und/oder Br substituiertes $C_6$-$C_{10}$-Aroyl oder einen Rest der Formeln (II), (IIIa) oder (IIIb)

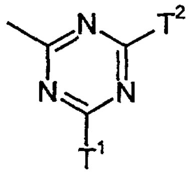
(II),

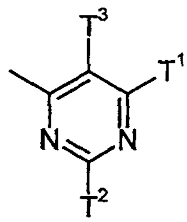
(IIIa),

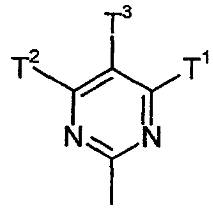
(IIIb),

worin

$T^1$ und $T^2$ unabhängig voneinander für $OR^5$, $NR^6 R^7$ oder $SR^8$ stehen,

worin

$R^5$ und $R^8$ unabhängig voneinander Wasserstoff, $C_1$-$C_6$-Alkyl, durch Sulfo, Carboxy, und/oder Di-$C_1$-$C_3$-alkylamino substituiertes $C_2$-$C_6$-Alkyl oder gegebenenfalls durch $C_1$-$C_4$-Alkyl, $C_1$-$C_4$-Alkoxy, Sulfo, F, Br und/oder Cl substituiertes Phenyl bedeuten,

$R^6$ Wasserstoff, $C_1$-$C_6$-Alkyl, gegebenenfalls durch Sulfo, Hydroxy, $C_1$-$C_3$-Alkoxy, Amino oder mono- oder disubstituiertes $C_1$-$C_3$-Alkylamino substituiertes $C_2$-$C_6$-Alkyl bedeutet,

$R^7$ unabhängig von $R^6$ die Bedeutung von $R^6$ besitzt oder gegebenenfalls durch $C_1$-$C_4$-Alkyl, $C_1$-$C_4$-Alkoxy, Carboxy, Sulfo, F, Cl und/oder Br substituiertes Phenyl oder Naphthyl bedeutet oder

$R^6$ und $R^7$ zusammen mit dem N-Atom, an das sie gebunden sind, einen Morpholinrest oder einen gegebenenfalls durch $C_1$-$C_4$-Alkyl, $C_2$-$C_4$-Hydroxyalkyl oder $C_2$-$C_4$-Aminoalkyl substituierten Piperazinrest bilden und

$T^3$ für Wasserstoff, F, Cl, CN, $CCl_3$, $CCl_2F$ oder COOH steht.

In einer weiteren besonderen Ausführungsform bedeutet

X im Falle n = 2
-CO-, -CO-$(CH_2)_{1-4}$-CO-, -$COC_2H_2$-CO- gegebenenfalls durch $CH_3$, $OCH_3$, F, Cl, Br und/oder COOH substituiertes $C_6$-$C_{10}$-Diaroyl oder einen Rest der Formeln (IVa), (IVb) oder (V)

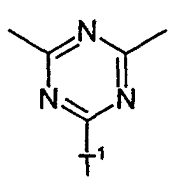
(IVa)

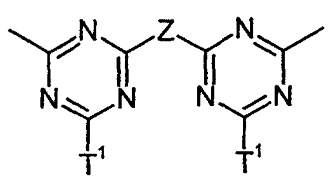
(IVb)

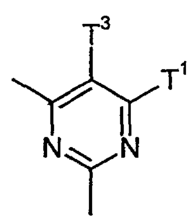
(V),

worin $T^1$ und $T^3$ die oben angegebene Bedeutung haben und

Z für einen Rest der Formel

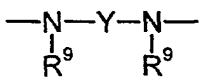 oder 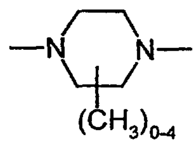

steht,

Y $C_2$-$C_6$-Alkylen oder gegebenenfalls Carboxy- oder Sulfogruppen-substituiertes Arylen bedeutet und

$R^9$ Wasserstoff, $C_1$-$C_6$-Alkyl oder $C_2$-$C_4$-Hydroxyalkyl bedeutet.

In einer ebenfalls besonderen Ausführungsform bedeutet

X im Falle n = 3 einen Rest der Formel (VI) oder (VII)

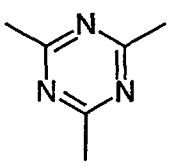 , 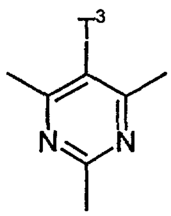

(VI) (VIII)

worin $T^3$ die oben angegebene Bedeutung besitzt.

Besonders bevorzugt sind Verbindungen der Formel (I), die der Formel (VIII) entsprechen

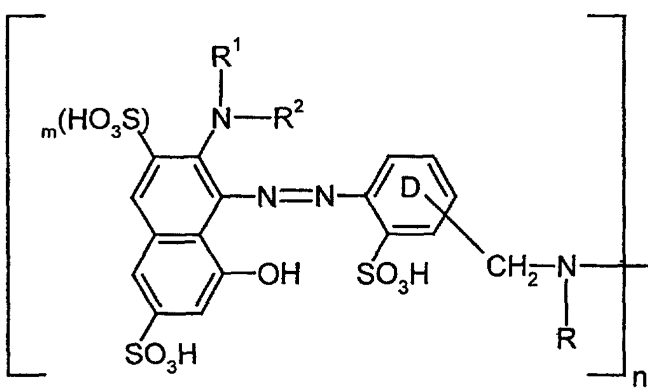 (VIII)

worin

R, $R^1$, $R^2$, D, X und m die oben angegebene Bedeutung haben,

n für 1 oder 2 steht.

Insbesondere sind dabei Farbstoffe der Formel (VIII) bevorzugt, worin m = 0 bedeutet.

Ganz besonders bevorzugt sind Farbstoffe der Formel (VIII) worin R, $R^1$ und $R^2$ die oben angegebene Bedeutung besitzen, m = 0 bedeutet, der Ring D keinen weiteren Substituenten mehr trägt und

X für den Fall n = 1, Benzoyl oder einen Rest der Formel (II) bedeutet und für den Fall n = 2, CO, $COC_2H_4CO$, $COC_2H_2CO$,

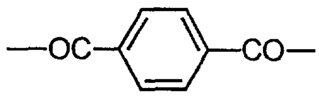

oder einen Rest der Formel (IVa) oder (IVb) bedeutet.

In einer bevorzugten Ausführungsform besitzen die Substituenten der Verbindung der Formel (I) und seiner Unterformeln folgende Bedeutungen:

$R^1$ und $R^2$ bedeuten unabhängig voneinander vorzugsweise Wasserstoff, $CH_3$, $C_2H_5$, $C_3H_7$, $C_2H_4OH$, $C_2H_4SO_3H$, $C_3H_6OH$, $C_3H_6SO_3H$, $C_2H_4COOH$, $C_3H_6COOH$, $C_6H_5$, $C_6H_4SO_3H$, $C_6H_4COOH$ oder $C_2H_4N(CH_3)_2$.

R bedeutet vorzugsweise Wasserstoff, $CH_3$, $C_2H_5$, $C_2H_4OH$.

$R^3$ und $R^4$ bedeuten unabhängig voneinander vorzugsweise Wasserstoff, $CH_3$, $C_2H_4OH$.

$R^5$ leitet sich vorzugsweise von folgenden Verbindungen $HOR^5$ ab: Ethylenglykol oder Isethionsäure (2-Hydroxyethansulfonsäure), Wasser.

$R^6$ bedeutet vorzugsweise Wasserstoff, $CH_3$, $C_2H_5$, $C_3H_7$, $C_2H_4OH$, $C_2H_4OCH_3$ oder $C_3H_6OH$.

$R^7$ bedeutet vorzugsweise Wasserstoff, $C_2H_4OH$, $C_3H_6OH$, $C_2H_4SO_3H$, $C_2H_4COOH$, $C_3H_6SO_3H$, $C_3H_6COOH$, $C_6H_5$, $C_6H_4SO_3H$, $C_6H_4COOH$ oder

$R^6$ und $R^7$ bilden zusammen mit dem N-Atom, an das sie gebunden sind vorzugsweise einen Rest der Formel

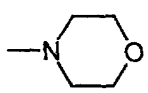 , 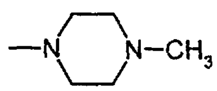 , 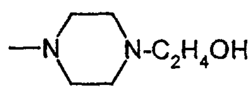 oder 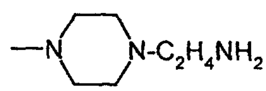

$R^8$ leitet sich vorzugsweise von folgenden Thiolen $HSR^8$ ab: 2-Mercaptoethanol oder 3-Mercapto-1-propansulfonsäure.

$R^9$ bedeutet vorzugsweise Wasserstoff, $CH_3$, $C_2H_4OH$.

$T^1$ und $T^2$ leiten sich unabhängig voneinander vorzugsweise von folgenden Aminen $T^1H$ bzw. $T^2H$ ab: 2-Aminoethanol, 2-Methylaminoethanol, 1-Amino-2-propanol, 2-Amino-1-propanol, Diethanolamin, Taurin, N-Methyltaurin, Glycin, 3-Aminopropansäure, 3-Dimethylamino-1-propylamin, N-2-Hydroxyethylpiperazin, N-2-Aminoethylpiperazin, Morpholin, Anilin, Orthanilsäure, Methanilsäure, Sulfanilsäure, Anthranilsäure, 3-Aminobenzoesäure, 4-Aminobenzoesäure, Ammoniak.

Z bedeutet vorzugsweise: m-Phenylendiamin, p-Phenylendiamin, 2,4-Diaminobenzolsulfonsäure, 2,5-Diaminobenzolsulfonsäure, Piperazin, 2,5-Dimethylpiperazin.

Ebenfalls ganz besonders bevorzugt sind Verbindungen der Formel (I), die der Formel (IX) entsprechen

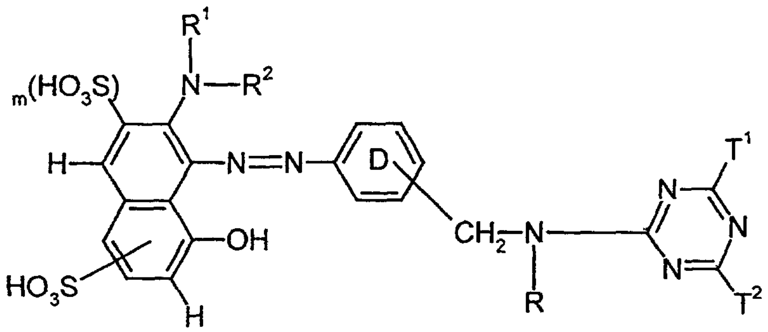

(IX),

worin die Substituenten die oben angegebene Bedeutungen besitzen.

Ganz besonders bevorzugt sind Verbindungen der Formel (X)

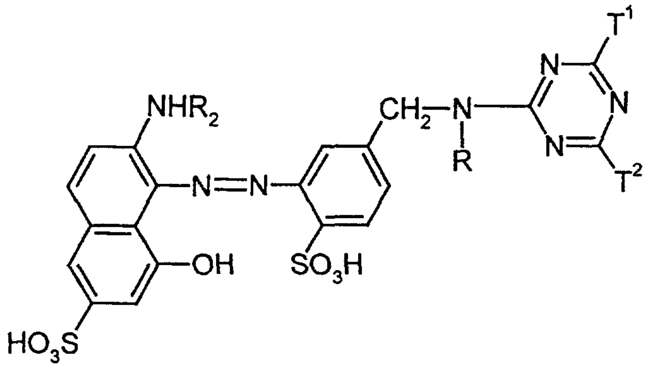

(X),

worin

R Wasserstoff, $CH_3$ oder $C_2H_5$ bedeutet,

$R^2$ für Wasserstoff, $CH_3$, $C_2H_5$, $C_3H_7$, $C_4H_9$ oder $C_6H_5$ steht und

$T^1$ und $T^2$ die oben angegebenen Bedeutungen haben, vorzugsweise für OH, $NH_2$, $NH_2C_2H_4OH$, $N(C_2H_4OH)_2$, $NHC_2H_4SO_3H$, $CH_2NC_2H_4SO_3H$ stehen.

Die Farbstoffe der Formel (I) werden bevorzugt in Form ihrer Lithium-, Natrium-, Kalium- oder Ammoniumsalze eingesetzt. Als Ammoniumsalze können insbesondere solche zum Einsatz kommen, bei denen 1 bis 4 Wasserstoffatome durch gleiche oder verschiedene $C_1$-$C_6$-Alkyl- oder durch Hydroxy- oder $C_1$-$C_3$-Alkoxygruppen substituierte $C_2$-$C_6$-Alkylreste ersetzt sind. Besonders bevorzugt sind Alkanolammoniumsalze wie Methyldiethanolammonium, Dimethylethanolammonium oder Triethanolammonium und quartäre Ammoniumionen wie Tetramethylammonium oder Tetraethylammonium.

Die Erfindung betrifft weiterhin ein Verfahren zur Herstellung der erfindungsgemäßen Verbindungen der Formel (I), das dadurch gekennzeichnet ist, daß man Verbindungen der Formel (XI)

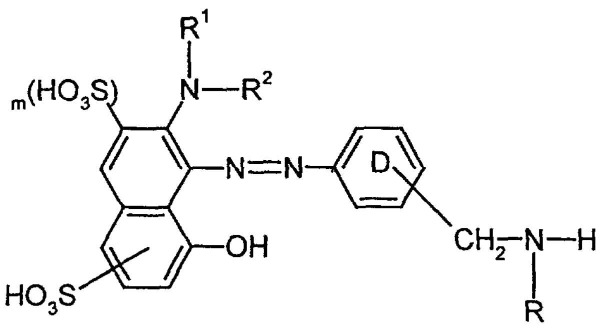

(XI)

worin

R, $R^1$, $R^2$, D und m die oben genannten Bedeutungen haben, mit Verbindungen der Formel

$$X(B)_n$$

umgesetzt, wobei

X in Abhängigkeit von n die obige Bedeutung hat,

n 1, 2 oder 3 bedeutet und

B ein abspaltbarer Cl oder F-Rest ist.

Als bevorzugte Verbindungen der Formel $X(B)_n$ werden Carbonsäurehalogenide oder -methylester, 2,4,6-Trifluor- oder 2,4,6-Trichlorpyrimidine oder -1,3,5-triazine eingesetzt und in diesen Umsetzungsprodukten gegebenenfalls in 4- und/oder 6-Stellung noch befindliche Fluor- bzw. Chloratome durch NH-, OH- oder SH-haltige Nukleophile substituiert.

Bevorzugte Acylierungsmittel der Formel $X(B)_n$ sind Carbonylchloride, z.B. Acetylchlorid, Propionylchlorid, Benzoylchlorid, Terephthaloylchlorid, Succinoylchlorid, Fumaroylchlorid, Phosgen und Carbonsäureanhydrid, z.B. Acetanhydrid.

Bevorzugte Pyrimidine der Formel $X(B)_n$ sind 2,4,6-Trifluorpyrimidin, 5-Chlor-2,4,6-trifluorpyrimidin, 5-Cyano-2,4,6-trifluorpyrimidin, 2,4,5,6-Tetrachlorpyrimidin.

Als Triazine der Formel $X(B)_n$ kommen bevorzugt 2,4,6-Trifluor- und 2,4,6-Trichlor-1,3,5-triazin in Frage.

Die Verbindungen der Formel (XI) sind bekannt: (CAS-RN 77850-13-6) oder können auf analoge Weise erhalten werden. Die Bedingungen für die Umsetzung von Verbindungen der Formel (XI) mit Acylierungsmitteln oder mit 2,4,6-Trifluor- oder 2,4,6-Trichlorpyrimidin oder -1,3,5-triazin sind ebenfalls bekannt (z.B. DE-A 29 35 681, EP-65 479).

Ebenso sind die Bedingungen für die Umsetzung von gegebenenfalls in 4- und/oder 6-Stellung noch in diesen Pyrimidin- oder Triazin-haltigen Umsetzungsprodukten vorhandenen Fluor- bzw. Chloratome mit NH-, OH- oder SH-haltigen Nukleophilen bekannt (EP-755 984, WO 96/24 635, EP-682 088).

Weiterer Gegenstand der Erfindung ist ein Verfahren zum Färben von cellulosehaltigen Materialien mit den Farbstoffen der Formel (I).

Die erfindungsgemäßen Verbindungen der Formel (I) können als feste oder flüssige Farbstoffpräparationen eingesetzt werden. Sie werden vorzugsweise in Form von wäßrigen Präparationen, insbesondere von Lösungen eingesetzt. Diese wäßrigen Farbstoffpräparationen enthalten im allgemeinen einen oder mehrere Farbstoffe der Formel (I), gegebenenfalls geeignete organische Lösungsmittel, worunter auch hydrotrope Verbindungen zählen können sowie weitere Hilfsmittel und/oder Stabilisatoren. Es ist vorteilhaft, die wäßrigen Farbstofflösungen im Zuge der Farbstoffsynthese selbst, ohne Zwischenisolierung des Farbstoffs, herzustellen.

Die Anwendungsform der wäßrigen Farbstoffpräparate ist insbesondere beim Färben oder Bedrucken von Papier bevorzugt. Die Herstellung einer stabilen, wäßrig konzentrierten Färbepräparation kann auf allgemeine Weise erfolgen, durch Lösen des Farbstoffs in Wasser gegebenenfalls unter Zugabe eines oder mehrerer Hilfsmittel, z.B. einer hydrotropen Verbindung oder eines Stabilisators.

Die wäßrigen Farbstoffpräparationen enthalten im allgemeinen etwa 0,5 bis 20 Gew.-% eines oder mehrerer Farbstoffe der Formel (I) und 80 bis 99,5 Gew.-% Wasser und/oder Lösungsmittel sowie gegebenenfalls weitere übliche

Bestandteile.

Bevorzugte organische Lösungsmittel sind dabei Alkohole und deren Ether oder Ester, Carbonsäureamide, Harnstoffe, Sulfoxide und Sulfone, insbesondere solche mit Molekulargewichten <200. Besonders geeignete Lösungsmittel sind beispielsweise: Methanol, Ethanol, Propanol; Ethylen-, Propylen-, Diethylen-, Thiodiethylenund Dipropylenglykol; Butandiol; β-Hydroxypropionitril, Pentamethylenglykol, Ethylenglykolmonoethyl- und propylether, Ethylendiglykolmonoethylether, Triethylenglykolmonobutylether, Butylpolyglykol, Formamid, Triethylenglykol, 1,5-Pentandiol, 1,3,6-Hexantriol, Essigsäure-2-hydroxyethylester, Essigsäure-2-(2'-hydroxy)-ethylester, Glycerin, Glykolacetat, 1,2-Dihydroxypropan, 1-Methoxy-2-propanol, 2-Methoxy-1-propanol, N,N-Dimethylformamid, Pyrrolidon, N-Methyl-caprolactam, ε-Caprolactam, Butyrolacton, Harnstoff, Tetramethylharnstoff, 1,3-Dimethyl-2-imidazolidinon, N,N'-Dimethylolpropylenharnstoff, Dimethylsulfoxid, Dimethylsulfon, Sulfolan, Isopropanol, Polyethylenglykol.

Als weitere für wäßrige Farbstoffpräparationen, insbesondere für Drucktinten übliche Zusätze kommen solche ionischen oder nichtionischen Stoffe in Frage, mit denen die Viskosität und/oder Oberflächenspannung in die für die Anwendung erforderlichen Bereiche eingestellt werden kann, wie beispielsweise anionische, kationische oder neutrale Tenside wie Dispergiermittel und Viskositätsregulatoren. Die Funktion von Viskositätsregulatoren kann beispielsweise von den organischen Lösungsmitteln übernommen werden.

Bevorzugt sind wäßrige Farbstoff-Präparationen, insbesondere Farbstoff-Lösungen, enthaltend

- 0,5 bis 20 Gew.-%, insbesondere 1 bis 15 Gew.-% eines oder mehrerer Farbstoffe, wobei mindestens einer der Formel (I) oder einem Salz davon entspricht,
- 50 bis 99,5 Gew.-%, insbesondere 85 bis 99 Gew.-% Wasser,
- 0 bis 30 Gew.-%, insbesondere 0 bis 20 Gew.-% eines oder mehrere organische Lösungsmittel,
- 0 bis 30 Gew.-%, insbesondere 0 bis 10 Gew.-% die Viskosität und/oder die Oberflächenspannung beeinflussende Zusätze,

wobei sich die Summe der genannten Inhaltsstoffe vorzugsweise auf 100 Gew.-% ergänzt.

Die wäßrigen Farbstoffpräparationen können durch Auflösen der Farbstoffsalze in Wasser oder aus den Kondensationslösungen, die gegebenenfalls einem Isomerenaustausch und/oder einer Entsalzung z.B. durch Druckpermeation unterworfen werden und/oder durch Zusatz eines oder mehrerer der obengenannten organischen Lösungsmitteln gegebenenfalls bei erhöhten Temperaturen (30 bis 100°C, insbesondere 30 bis 50°C) und unter Zusatz von anorganischen und organischen Basen hergestellt werden; gegebenenfalls können zusätzlich noch übliche ionische oder nichtionische Zusatzstoffe verwendet werden, z.B. solche, mit denen die Viskosität erniedrigt und/oder die Oberflächenspannung erhöht werden können.

Anstelle der Salze von (I) können auch die entsprechenden freien Säuren in Kombination mit mindestens äquimolaren Mengen der entsprechenden organischen oder anorganischen Basen eingesetzt werden.

Die erfindungsgemäßen wäßrigen Farbstoffpräparationen eignen sich weiterhin zur Herstellung von Drucktinten, die insbesondere auch als Aufzeichnungsflüssigkeiten nach der Ink-Jet-Methode eingesetzt werden können.

Weiterer Gegenstand der Erfindung sind daher Drucktinten enthaltend mindestens einen Farbstoff (I) sowie deren Verwendung als Aufzeichnungsflüssigkeit für Ink-Jet-Aufzeichnungssysteme zur Erzeugung roter Drucke.

Unter der Ink-Jet-Methode des erfindungsgemäßen Verfahrens wird ein Tintenstrahlaufzeichnungsverfahren verstanden, bei dem die Tintentropfen auf das Substrat gespritzt werden. Die feinen Tintentröpfchen können durch unterschiedliche Verfahren erzeugt werden. Bevorzugt werden sie nach den allgemein bekannten Thermal-Jet, Bubble-Jet, Piezzo-Jet oder Ventil-Ink-Jet-Verfahren erzeugt.

Bei der Verwendung der erfindungsgemäßen Farbstoffe in Form ihrer wäßrigen Präparationen, insbesondere ihrer Drucktinten als Aufzeichnungsflüssigkeit für Ink-Jet-Aufzeichnungssysteme ergeben sich folgende Vorteile: Die physikalischen Eigenschaften, wie Viskosität, Oberflächenspannung und dergleichen, liegen in den geeigneten Bereichen; die Aufzeichnungsflüssigkeit verursacht keine Verstopfungen in feinen Abgabeöffnungen von Tintenstrahl-Aufzeichnungsvorrichtungen; sie liefert Bilder von hoher Dichte; bei der Lagerung kommt es in der Aufzeichnungsflüssigkeit nicht zu einer Veränderung von physikalischen Eigenschaften und zur Ablagerung von festen Bestandteilen; die Aufzeichnungsflüssigkeit eignet sich zur Aufzeichnung auf verschiedenen Aufzeichnungsmedien ohne Beschränkungen hinsichtlich der Art der Aufzeichnungsmedien; schließlich fixiert die Aufzeichnungsflüssigkeit rasch und ergibt Bilder mit ausgezeichneter Wasserfestigkeit, Lichtechtheit, Abriebbeständigkeit und Auflösung.

Die folgenden Herstellungsbeispiele sollen die vorliegende Erfindung erläutern ohne sie jedoch darauf zu beschränken. In den Beispielen bedeuten Teile stets Gewichtsteile, falls nichts anderes angegeben ist.

Die mit den erfindungsgemäßen Farbstoffen erhaltenen Färbungen bzw. Drucke, insbesondere die Drucke, die nach der Ink-Jet-Methode auf Papier erhalten wurden, weisen eine hervorragende Brillanz auf.

**Beispiele**

**Beispiel 1**

48,0 g der Verbindung der Formel

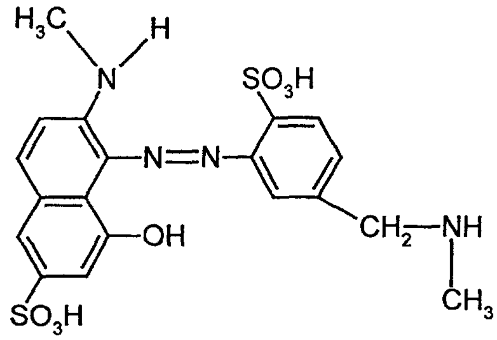

werden mit Hilfe von Soda in 1000 ml Wasser neutral gelöst. Nach Zugabe vor 19 g 2,4,6-Trichlor-1,3,5-triazin wird der Ansatz bei 0 - 5°C gerührt, wobei der pH-Wert durch Zugabe von Soda-Lösung bei 7,5 gehalten wird. Nach Beendigung der Kondensation werden 13 g Taurin zugesetzt; der Ansatz wird auf 40°C erwärmt, dabei wird der pH-Wert durch Zugabe von Soda-Lösung bei 7,5 - 8 gehalten. Nach Beendigung der zweiten Kondensation werden 21 g Diethanolamin zugesetzt; der Ansatz wird zum Sieden erhitzt, bis die dritte Kondensation beendet ist.

Die resultierende Lösung des Farbstoffes der Formel

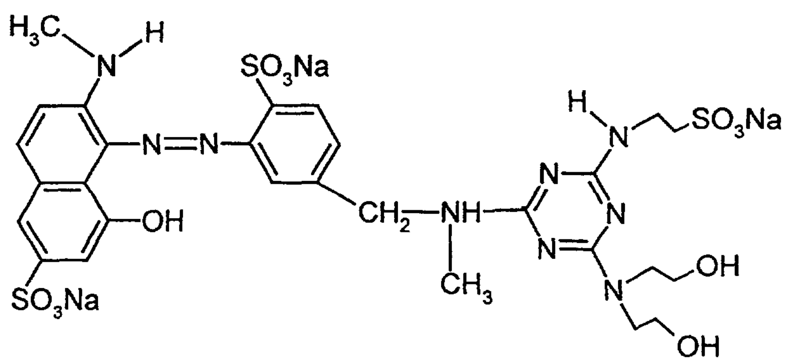

wird in einer Druckpermeationsanlage weitgehend entsalzt und am Ende konzentriert, bis der $E_1^1$-Wert des Absorptionsmaximums 40 ist ($\lambda_{max}$ = 539 nm).

$E_1^1$-Wert: normierte Extinktion einer 1 %igen Lösung (Wasser) bei einer Schichtdicke von 1 cm.

**Beispiel 2**

10 g der konzentrierten Lösung aus Beispiel 1 werden mit 70 g Wasser und 20 g 1,5-Pentandiol verrührt. Man erhält eine Tinte, die beim Verdrucken mit einem handelsüblichen Ink-Jet-Drucker rote, brillante, lichtechte Aufzeichnungen auf handelsüblichen Papieren liefert.

In der folgenden Tabelle sind weitere Farbstoffe aufgeführt, zu deren Herstellung die angegebenen Verbindungen für die zweite und dritte Kondensation an 2,4,6-Trichlor-1,3,5-triazin eingesetzt werden. Bei Verwendung von 3-Mercaptopropansulfonsäure sowie von 2-Mercaptoethanol wird außerdem die zur Deprotonierung der SH-Gruppe benötigte Menge Soda zugesetzt.

| Beispiel | 2. Kondensation | 3. Kondensation |
|---|---|---|
| 3 | N-Methyltaurin | Ethanolamin |

(fortgesetzt)

| Beispiel | 2. Kondensation | 3. Kondensation |
|---|---|---|
| 4 | 3-Mercaptopropansulfonsäure | Natronlauge |
| 5 | Anthranilsäure | Natronlauge |
| 6 | Metanilsäure | Morpholin |
| 7 | 3-Mercaptopropansulfonsäure | 3-Mercaptopropansulfonsäure |
| 8 | Anthranilsäure | Diethanolamin |
| 9 | 3-Mercaptopropansulfonsäure | Diethanolamin |

Die resultierenden Lösungen enthalten die Farbstoffe der Formeln

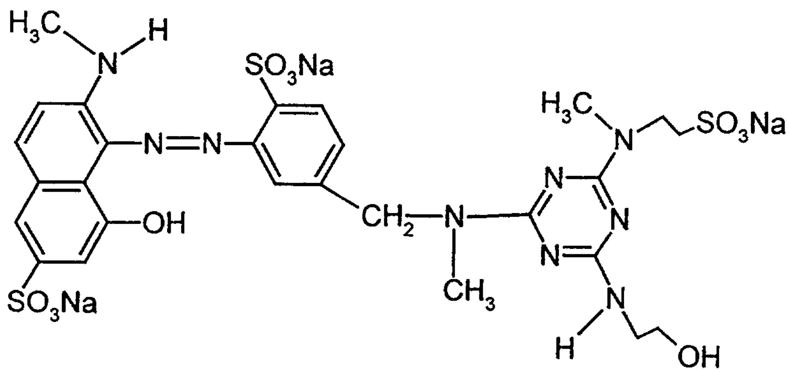

Beispiel 3

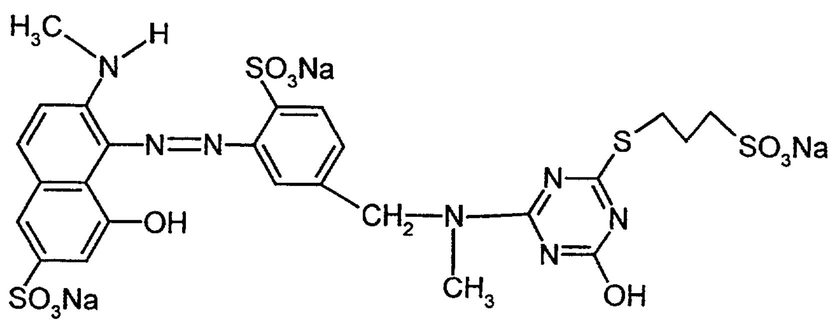

Beispiel 4

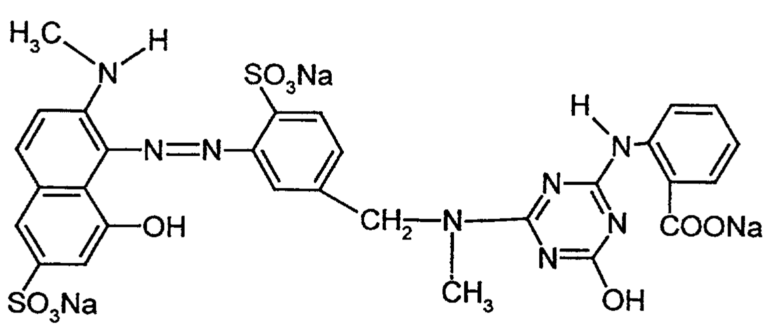

Beispiel 5

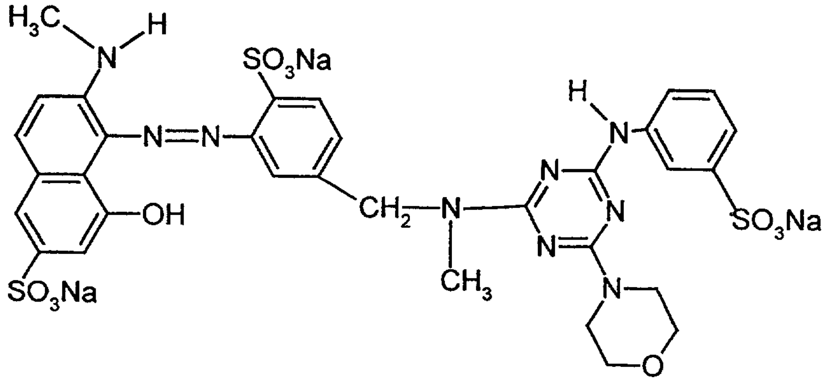

Beispiel 6

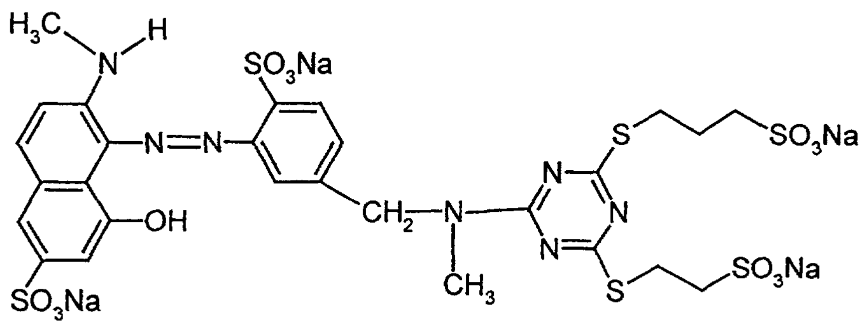

Beispiel 7

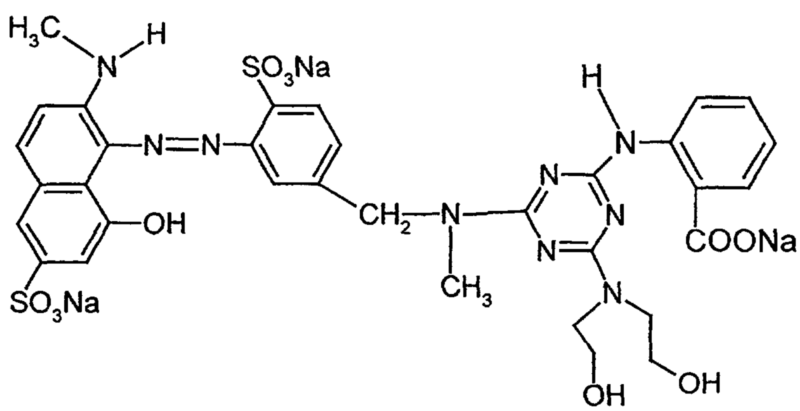

Beispiel 8

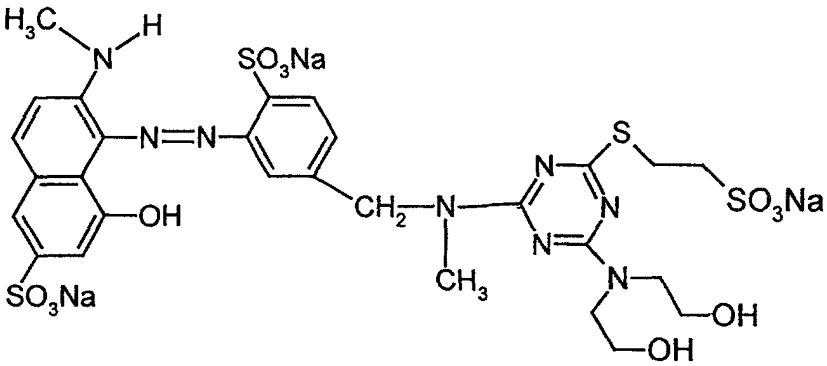

Beispiel 9

Verwendet man statt der in Beispiel 7 eingesetzten 3-Mercaptopropansulfonsäure equimolare Mengen Ethanolamin, Diethanolamin, N-Methyl-ethanolamin, Taurin, N-Methyltaurin, Morpholin, N-2-Hydroxyethylpiperazin, 2-Mercaptoethanol, Ammoniak oder Natronlauge, so erhält man ebenfalls Lösungen roter Farbstoffe.

**Beispiel 10**

Verwendet man statt des in Beispiel 1 eingesetzten Taurins weitere 48 g der in der ersten Kondensation eingesetzten Azoverbindung, so erhält man eine Lösung des Farbstoffs der Formel

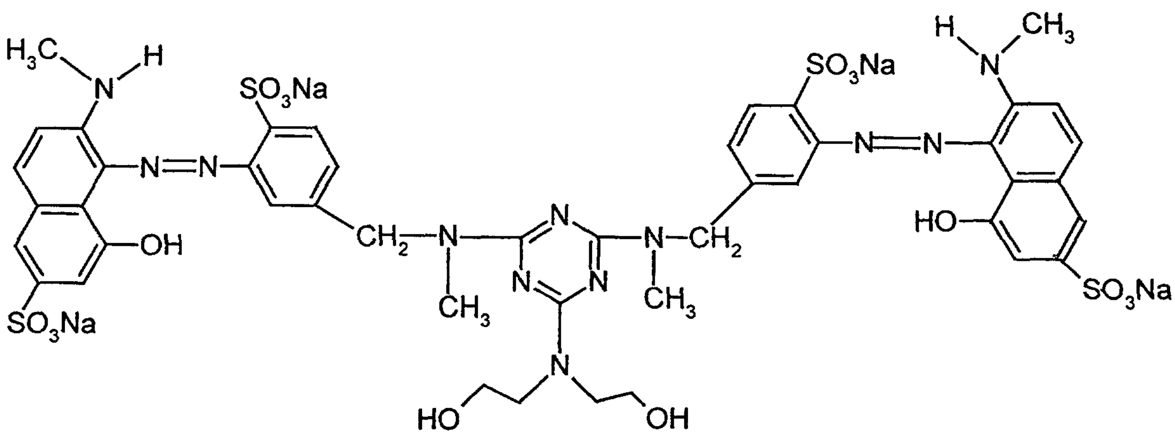

**Beispiel 11**

Verwendet man statt des in Beispiel 10 eingesetzten Diethanolamins eine equimolare Menge Natriumhydroxid, so erhält man eine Lösung des Farbstoffes der Formel

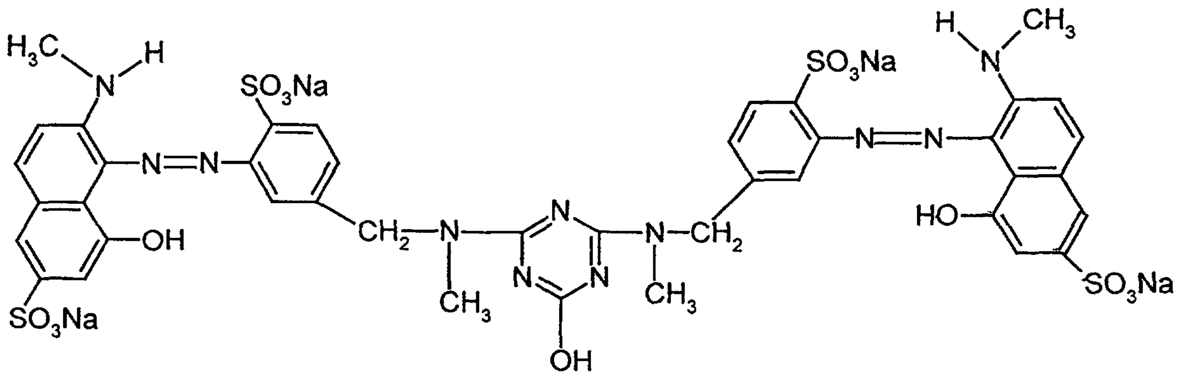

Durch die Verwendung von Ethanolamin, Taurin, N-Methyltaurin, Morpholin, N-2-Hydroxyethylpiperazin, 2-Mercaptoethanol, 3-Mercapto-propansulfonsäure oder Ammoniak anstelle von Natriumhydroxid erhält man die entsprechenden Farbstoffe, bei deren Verwendung in Ink-jet-Tinten man ebenfalls brillante, lichtechte rote Drucke erhält.

**Beispiel 12**

48 g der Azoverbindung aus Beispiel 1 werden in 1000 ml Wasser mit Hilfe von Lithiumcarbonat neutral gelöst. Nach Zugabe von 10,1 g Terephthaloylchlorid wird der Ansatz bei 20 bis 25°C gerührt, wobei der pH-Wert durch Zugabe von Lithiumhydroxid-Lösung bei 7,5 bis 8 gehalten wird. Man erhält eine Lösung des Farbstoffs der Formel

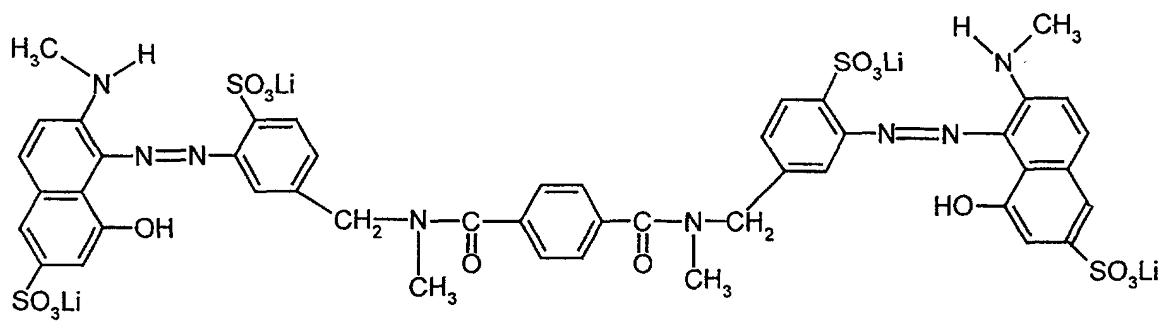

**Beispiel 13**

Verwendet man statt der in Beispiel 1 eingesetzten Menge Taurin 5,2 1,4-Diaminobenzol und statt des Diethanolamins eine equimolare Menge Taurin, so erhält man den roten Farbstoff der Formel

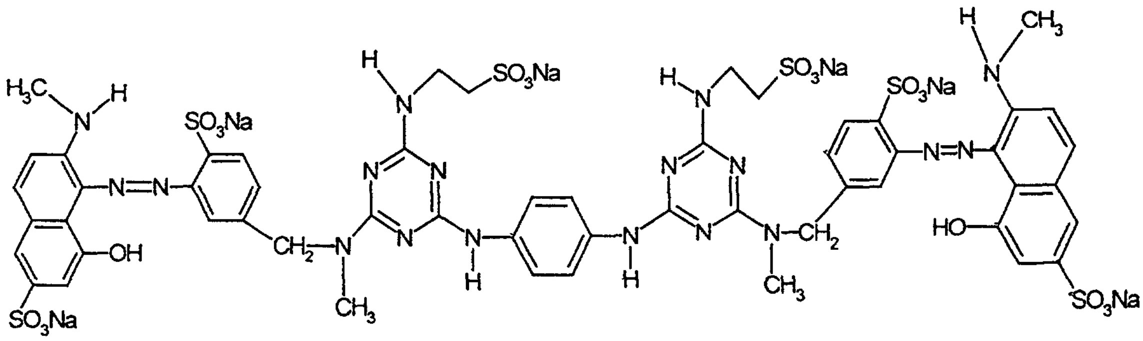

In der folgenden Tabelle sind weitere Farbstoffe aufgeführt, zu deren Herstellung die angegebenen Verbindungen für die zweite und dritte Kondensation an 2,4,6-Trichlor-1,3,5-triazin eingesetzt werden.

| Beispiel | 2. Kondensation | 3. Kondensation |
|---|---|---|
| 14 | 1,3-Diaminobenzol | N-Methyltaurin |
| 15 | 2,5-Dimethylpiperazin | Natronlauge |
| 16 | 2,5-Diaminobenzolsulfonsäure | Diethanolamin |
| 17 | 4,4'-Diaminostilben-2,2'-disulfonsäure | Ethanolamin |

## Patentansprüche

**1.** Verbindungen der Formel (I)

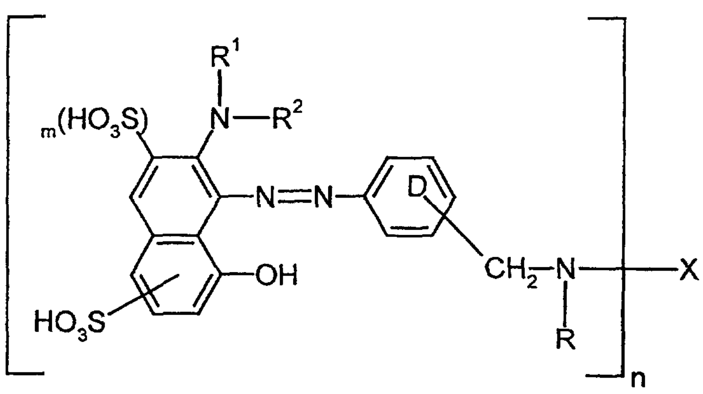

(I),

worin

R für Wasserstoff oder gegebenenfalls substituiertes $C_1$-$C_6$-Alkyl steht,

$R^1$ Wasserstoff oder $C_1$-$C_6$-Alkyl bedeutet,

$R^2$ Wasserstoff, gegebenenfalls substituiertes $C_1$-$C_6$-Alkyl oder gegebenenfalls substituiertes Phenyl bedeutet,

der Ring D gegebenenfalls durch ein oder mehrere gleiche oder verschiedene Substituenten aus der Reihe $C_1$-$C_6$-Alkyl, $C_1$-$C_6$-Alkoxy, Halogen, insbesondere F, Cl und Br und $SO_3H$ substituiert ist,

m 0 oder 1 bedeutet,

n 1, oder 3 bedeutet und in Abhängigkeit von n

X für einen mono-, di- oder trivalenten Acylrest, Pyrimidin- oder 1,3,5-Triazinrest steht, der nicht faserreaktiv ist.

**2.** Verbindungen gemäß Anspruch 1, worin

R für Wasserstoff, $C_1$-$C_6$-Alkyl oder $C_2$-$C_4$-Hydroxyalkyl steht,

$R^1$ Wasserstoff oder $C_1$-$C_6$-Alkyl bedeutet,

$R^2$ Wasserstoff, $C_1$-$C_6$-Alkyl, $C_2$-$C_4$-Hydroxyalkyl, $C_2$-$C_4$-Aminoalkyl, insbesondere durch $NR^3R^4$ substituiertes $C_2$-$C_4$-Alkyl bedeutet, worin

$R^3$ und $R^4$ unabhängig voneinander für Wasserstoff, $C_1$-$C_4$-Alkyl und $C_2$-$C_4$-Hydroxyalkyl stehen, oder gegebenenfalls durch $C_1$-$C_4$-Alkyl, $C_1$-$C_4$-Alkoxy, COOH, $SO_3H$, F, Cl und/oder Br substituiertes Phenyl bedeuten und

der Ring D gegebenenfalls substituiert ist durch einen oder mehrere gleiche oder verschiedene Substituenten aus der Reihe $C_1$-$C_4$-Alkyl, $C_1$-$C_4$-Alkoxy, F, Cl und Br.

**3.** Verbindungen gemäß Anspruch 1, **dadurch gekennzeichnet, daß**

X im Falle n = 1 gegebenenfalls durch COOH substituiertes $C_2$-$C_6$-Alkanoyl, gegebenenfalls durch $C_1$-$C_4$-Alkyl, $C_1$-$C_4$-Alkoxy, $SO_3H$, COOH, F, Cl und/oder Br substituiertes $C_6$-$C_{10}$-Aroyl oder einen Rest der Formeln (II), (IIIa) oder (IIIb)

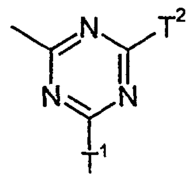
(II),

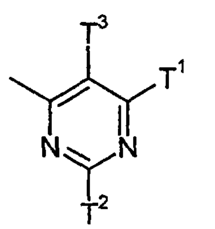
(IIIa),

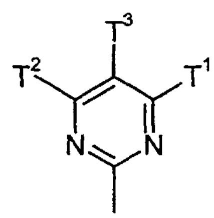
(IIIb),

worin

$T^1$ und $T^2$ unabhängig voneinander für $OR^5$, $NR^6R^7$ oder $SR^8$,

worin

$R^5$ und $R^8$ unabhängig voneinander Wasserstoff, $C_1$-$C_6$-Alkyl, durch Sulfo, Carboxy, und/oder Di-$C_1$-$C_3$-alkylamino substituiertes $C_2$-$C_6$-Alkyl oder gegebenenfalls durch $C_1$-$C_4$-Alkyl, $C_1$-$C_4$-Alkoxy, Sulfo, F, Br und/oder Cl substituiertes Phenyl bedeuten,

$R^6$ Wasserstoff, $C_1$-$C_6$-Alkyl oder gegebenenfalls durch Sulfo, Hydroxy, $C_1$-$C_3$-Alkoxy, Amino oder mono- oder disubstituiertes $C_1$-$C_3$-Alkylamino substituiertes $C_2$-$C_6$-Alkyl bedeutet,

$R^7$ unabhängig von $R^6$ die Bedeutung von $R^6$ besitzt oder gegebenenfalls durch $C_1$-$C_4$-Alkyl, $C_1$-$C_4$-Alkoxy, Carboxy, Sulfo, F, Cl und/oder Br substituiertes Phenyl oder Naphthyl bedeutet oder

$R^6$ und $R^7$ zusammen mit dem N-Atom, an das sie gebunden sind, einen Morpholinrest oder einen gegebenen-

falls durch $C_1$-$C_4$-Alkyl, $C_2$-$C_4$-Hydroxyalkyl oder $C_2$-$C_4$-Aminoalkyl substituierten Piperazinrest bilden und

$T^3$ für Wasserstoff, F, Cl, CN, $CCl_3$, $CCl_2F$ oder COOH steht,

X im Falle n = 2
-CO-, -CO-$(CH_2)_{1-4}$-CO-, -$COC_2H_2$-CO- gegebenenfalls durch $CH_3$, $OCH_3$, F, Cl, Br und/oder COOH substituiertes $C_6$-$C_{10}$-Diaroyl oder einen Rest der Formeln (IVa), (IVb) oder (V)

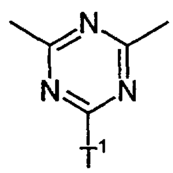

(IVa),

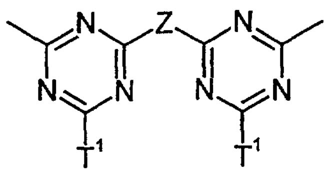

(IVb),

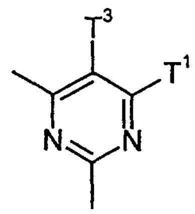

(V),

worin $T^1$ und $T^3$ die oben angegebene Bedeutung haben und

Z für einen Rest der Formel

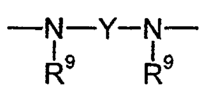

oder

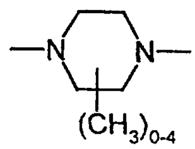

steht,

Y $C_2$-$C_6$-Alkylen oder gegebenenfalls Carboxy- oder Sulfatogruppensubstituiertes Arylen bedeutet,

$R^9$ Wasserstoff, $C_1$-$C_6$-Alkyl oder $C_2$-$C_4$-Hydroxyalkyl bedeutet,

X im Falle n = 3 einen Rest der Formel (VI) oder (VII)

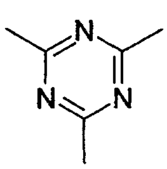

,

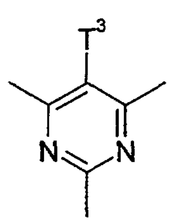

(VI) (VIII)

worin $T^3$ die oben angegebene Bedeutung besitzt.

**4.** Verbindungen gemäß Anspruch 1, **dadurch gekennzeichnet, daß** sie der Formel (VIII) entsprechen

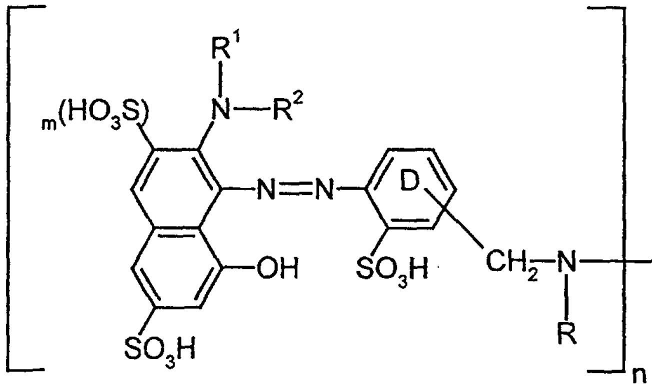

(VIII),

worin

| | |
|---|---|
| R, $R^1$, $R^2$, D, X und m | die oben angegebene Bedeutung haben, |
| n | für 1 oder 2 steht. |

**5.** Verbindungen gemäß Anspruch 1, **dadurch gekennzeichnet, daß**

| | |
|---|---|
| $R^1$ | Wasserstoff, $CH_3$, $C_2H_5$ oder $C_3H_7$ bedeutet, |
| $R^2$ | bedeutet vorzugsweise Wasserstoff, $CH_3$, $C_2H_5$, $C_3H_7$, $C_2H_4OH$, $C_2H_4SO_3H$, $C_3H_6OH$, $C_3H_6SO_3H$, $C_2H_4COOH$, $C_3H_6COOH$, $C_6H_5$, $C_6H_4SO_3H$, $C_6H_4COOH$ oder $C_2H_4N(CH_3)_2$, |
| R | bedeutet vorzugsweise Wasserstoff, $CH_3$, $C_2H_5$, $C_2H_4OH$, |
| $R^3$ und $R^4$ | bedeuten unabhängig voneinander vorzugsweise Wasserstoff, $CH_3$, $C_2H_4OH$, |
| $R^5$ | leitet sich vorzugsweise von folgenden Verbindungen $HOR^5$ ab: Ethylenglykol oder Isethionsäure (2-Hydroxyethansulfonsäure), |
| $R^6$ | bedeutet vorzugsweise Wasserstoff, $CH_3$, $C_2H_5$, $C_3H_7$, $C_2H_4OH$, $C_2H_4OCH_3$ oder $C_3H_6OH$, |
| $R^7$ | bedeutet vorzugsweise Wasserstoff, $C_2H_4OH$, $C_3H_6OH$, $C_2H_4SO_3H$, $C_2H_4COOH$, $C_3H_6SO_3H$, $C_3H_6COOH$, $C_6H_5$, $C_6H_4SO_3H$, $C_6H_4COOH$ oder |
| $R^6$ und $R^7$ | bilden zusammen mit dem N-Atom, an das sie gebunden sind vorzugsweise einen Rest der Formel |

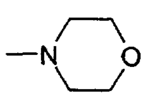 , 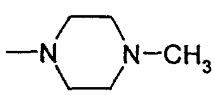 , 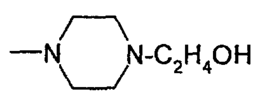 oder 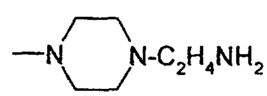

| | |
|---|---|
| $R^8$ | leitet sich vorzugsweise von folgenden Thiolen $HSR^8$ ab: 2-Mercaptoethanol oder 3-Mercapto-1-propansulfonsäure, |
| $R^9$ | bedeutet vorzugsweise Wasserstoff, $CH_3$, $C_2H_4OH$. |

**6.** Verbindungen gemäß Anspruch 1, **dadurch gekennzeichnet, daß** sie der Formel (IX) entsprechen

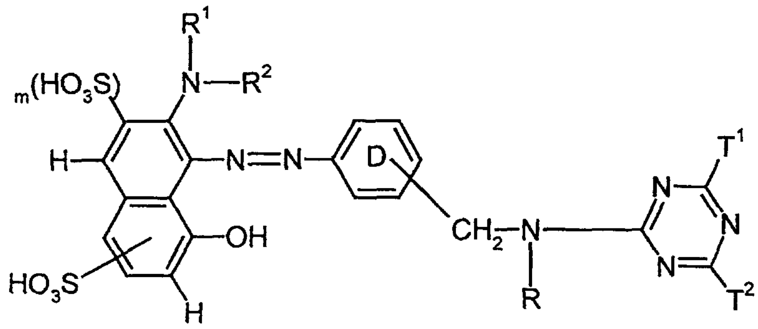

(IX),

worin die Substituenten die oben angegebene Bedeutungen besitzen.

**7.** Verbindungen gemäß Anspruch 1, **dadurch gekennzeichnet, daß** sie der Formel (X) entsprechen

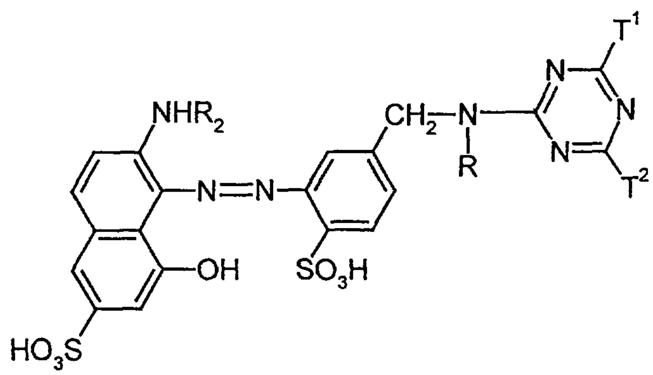

(X),

worin

R Wasserstoff, $CH_3$ oder $C_2H_5$ bedeutet,

$R^2$ für Wasserstoff, $CH_3$, $C_2H_5$, $C_3H_7$, $C_4H_9$ oder $C_6H_5$ steht und

$T^1$ und $T^2$ die oben angegebenen Bedeutungen haben.

**8.** Verfahren zur Herstellung von Verbindungen gemäß Anspruch 1, **dadurch gekennzeichnet, daß** man Verbindungen der Formel (XI)

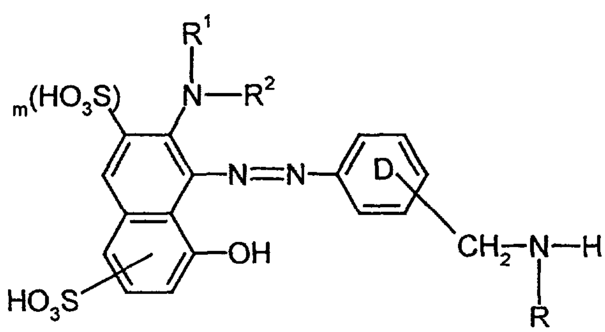

(XI),

worin

R, $R^1$, $R^2$, D und m die oben genannten Bedeutungen haben mit Verbindungen der Formel

$$X(B)_n$$

umsetzt, wobei

X in Abhängigkeit von n die obige Bedeutung hat,

n 1, 2 oder 3 bedeutet und

B ein abspaltbarer Cl oder F-Rest ist.

**9.** Wäßrige Farbstoff-Präparationen, insbesondere Farbstoff-Lösungen, enthaltend

- 0,5 bis 20 Gew.-% eines oder mehrerer Farbstoffe, wobei mindestens einer davon der Verbindung des Anspruchs 1 oder einem Salz davon entspricht,
- 50 bis 99,5 Gew.-% Wasser,
- 0 bis 30 Gew.-% eines oder mehrerer organische Lösungsmittel,
- 0 bis 30 Gew.-% die Viskosität und/oder die Oberflächenspannung beeinflussende Zusätze.

**10.** Drucktinten enthaltend mindestens einen Farbstoff, der einer Verbindung gemäß Anspruch 1 entspricht.

**11.** Verwendung der Verbindungen des Anspruchs 1 zum Färben und Bedrucken von hydroxy- und/oder amidgruppenhaltigen Substraten, insbesondere zum Bedrucken von Papier nach der Ink-Jet-Methode.

**Claims**

**1.** Compounds of the formula (I)

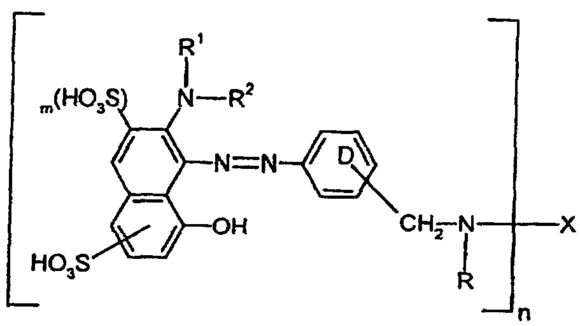

(I),

where

R is hydrogen or optionally substituted $C_1$-$C_6$-alkyl,

$R^1$ is hydrogen or $C_1$-$C_6$-alkyl,

$R^2$ is hydrogen, optionally substituted $C_1$-$C_6$-alkyl or optionally substituted phenyl,

the ring D is optionally substituted by one or more identical or different substituents selected from the group

consisting of $C_1$-$C_6$-alkyl, $C_1$-$C_6$-alkoxy, halogen, especially F, Cl and Br, and $SO_3H$,

m is 0 or 1,

n is 1, 2 or 3 and

X is as a function of n a mono-, di- or trivalent acyl, pyrimidine or 1,3,5-triazine radical which is not fibre reactive.

**2.** Compounds according to Claim 1, wherein

R is hydrogen, $C_1$-$C_6$-alkyl or $C_2$-$C_4$-hydroxyalkyl,

$R^1$ is hydrogen or $C_1$-$C_6$-alkyl,

$R^2$ is hydrogen, $C_1$-$C_6$-alkyl, $C_2$-$C_4$-hydroxyalkyl, $C_2$-$C_4$-aminoalkyl, especially $NR^3R^4$-substituted $C_2$-$C_4$-alkyl, where

$R^3$ and $R^4$ are independently hydrogen, $C_1$-$C_4$-alkyl and $C_2$-$C_4$-hydroxyalkyl or optionally $C_1$-$C_4$-alkyl-, $C_1$-$C_4$-alkoxy-, COOH-, $SO_3H$-, F-, Cl- and/or Br-substituted phenyl, and

the ring D is optionally substituted by one or more identical or different substituents selected from the group consisting of $C_1$-$C_4$-alkyl, $C_1$-$C_4$-alkoxy, F, Cl and Br.

**3.** Compounds according to Claim 1, **characterized in that**

X in the case where n = 1 is optionally COOH-substituted $C_2$-$C_6$-alkanoyl, optionally $C_1$-$C_4$-alkyl-, $C_1$-$C_4$-alkoxy-, $SO_3H$-, COOH-, F-, Cl- and/or Br-substituted $C_6$-$C_{10}$-aroyl or a radical of the formulae (II), (IIIa) or (IIIb)

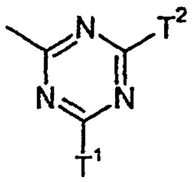

(II),

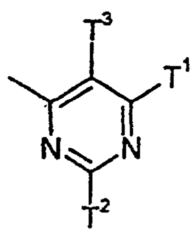

(IIIa),

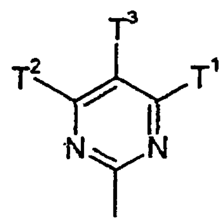

(IIIb),

where

$T^1$ and $T^2$ are independently $OR^5$, $NR^6R^7$ or $SR^8$,

where

$R^5$ and $R^8$ are independently hydrogen, $C_1$-$C_6$-alkyl, sulphur-, carboxyl- and/or di-$C_1$-$C_3$-alkylamino-substituted $C_2$-$C_6$-alkyl or optionally $C_1$-$C_4$-alkyl-, $C_1$-$C_4$-alkoxy-, sulphur-, F-, Br- and/or Cl-substituted phenyl,

$R^6$ is hydrogen, $C_1$-$C_6$-alkyl or is $C_2$-$C_6$-alkyl optionally substituted by sulphur, hydroxyl, $C_1$-$C_3$-alkoxy, amino or mono- or disubstituted $C_1$-$C_3$-alkylamino,

$R^7$ has the meaning of $R^6$ independently of $R^6$ or is optionally $C_1$-$C_4$-alkyl-, $C_1$-$C_4$-alkoxy-, carboxyl-, sulphur-, F-, Cl- and/or Br-substituted phenyl or naphthyl, or

$R^6$ and $R^7$ combine with the nitrogen atom to which they are attached to form a morpholine radical or an optionally $C_1$-$C_4$-alkyl-, $C_2$-$C_4$-hyrdoxyalkyl- or $C_2$-$C_4$-aminoalkyl-substituted piperazine radical, and

$T^3$ is hydrogen, F, Cl, CN, $CCl_3$, $CCl_2F$ or COOH,

X in the case where n = 2 is $-CO-$, $-CO-(CH_2)_{1-4}-CO-$, $-COC_2H_2-CO-$, optionally $CH_3$-, $OCH_3$-, F-, Cl-, Br- and/or COOH-substituted $C_6$-$C_{10}$-diaroyl or a radical of the formulae (IVa), (IVb) or (V)

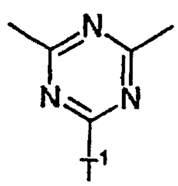
(IVa),

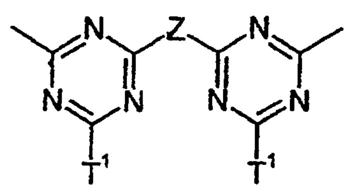
{IVb),

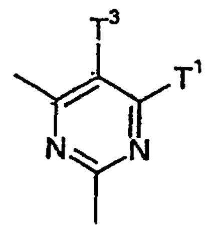
(V),

where $T^1$ and $T^3$ are each as defined above and

Z is a radical of the formula

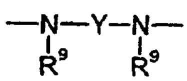
or
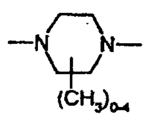

Y is $C_2$-$C_6$-alkylene or optionally carboxyl- or sulphato-substituted arylene,

$R^9$ is hydrogen, $C_1$-$C_6$-alkyl or $C_2$-$C_4$-hydroxyalkyl, and

X in the case where n = 3 is a radical of the formula (VI) or (VII)

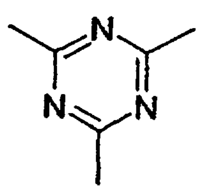
,
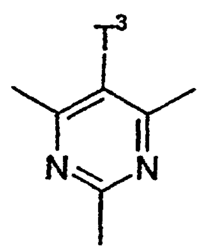

(VI) (VIII)

where $T^3$ is as defined above.

**4.** Compounds according to Claim 1, **characterized in that** they conform to the formula (VIII)

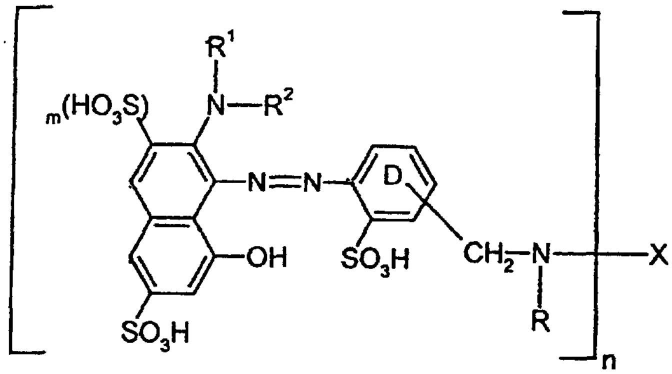

(VIII),

where

| | |
|---|---|
| R, $R^1$, $R^2$, D, X and m | are as defined above and |
| n | is 1 or 2. |

**5.** Compounds according to Claim 1, **characterized in that**

| | |
|---|---|
| $R^1$ | is hydrogen, $CH_3$, $C_2H_5$ or $C_3H_7$, |
| $R^2$ | is preferably hydrogen, $CH_3$, $C_2H_5$, $C_3H_7$, $C_2H_4OH$, $C_2H_4SO_3H$, $C_3H_6OH$, $C_3H_6SO_3H$, $C_2H_4COOH$, $C_3H_6COOH$, $C_6H_5$, $C_6H_4SO_3H$, $C_6H_4COOH$ or $C_2H_4N(CH_3)_2$, |
| R | is preferably hydrogen, $CH_3$, $C_2H_5$, $C_2H_4OH$, |
| $R^3$ and $R^4$ | are independently preferably hydrogen, $CH_3$ or $C_2H_4OH$, |
| $R^5$ | is preferably derived from the following $HOR^5$ compounds: ethylene glycol or isethionic acid (2-hydroxyethanesulphonic acid), |
| $R^6$ | is preferably hydrogen, $CH_3$, $C_2H_5$, $C_3H_7$, $C_2H_4OH$, $C_2H_4OCH_3$ or $C_3H_6OH$, |
| $R^7$ | is preferably hydrogen, $C_2H_4OH$, $C_3H_6OH$, $C_2H_4SO_3H$, $C_2H_4COOH$, $C_3H_6SO_3H$, $C_3H_6COOH$, $C_6H_5$, $C_6H_4SO_3H$, $C_6H_4COOH$, or |
| $R^6$ and $R^7$ | combine with the nitrogen atom to which they are attached to preferably form a radical of the formula |

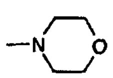 , 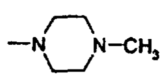 , 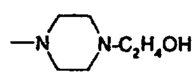 or 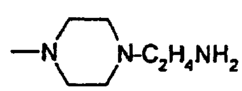

| | |
|---|---|
| $R^8$ | is preferably derived from the following $HSR^8$ thiols: 2-mercaptoethanol or 3-mercapto-1-propanesulphonic acid, |
| $R^9$ | is preferably hydrogen, $CH_3$ or $C_2H_4OH$. |

**6.** Compounds according to Claim 1, **characterized in that** they conform to the formula (IX)

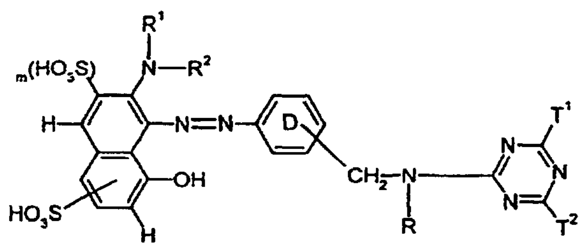

(IX),

where the substituents are each as defined above.

**7.** Compounds according to Claim I, **characterized in that** they conform to the formula (X)

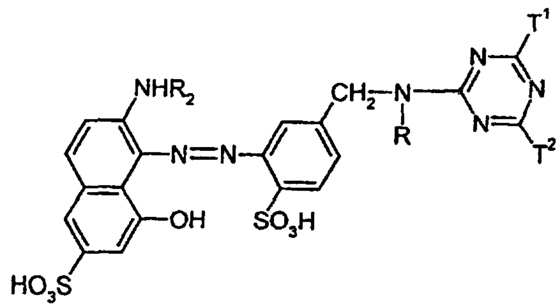

(X),

where

R is hydrogen, $CH_3$ or $C_2H_5$

$R^2$ is hydrogen, $CH_3$, $C_2H_5$, $C_3H_7$, $C_4H_9$ or $C_6H_5$ and

$T^1$ and $T^2$ are each as defined above.

**8.** Process for preparing compounds according to Claim 1, **characterized in that** compounds of the formula (XI)

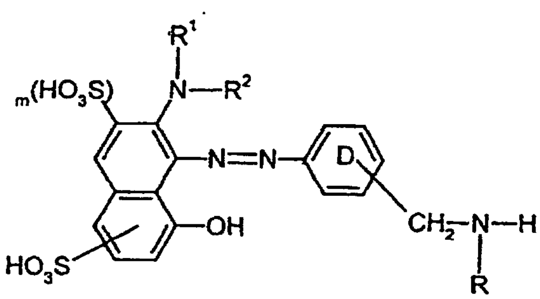

(XI),

where
R, $R^1$, $R^2$, D and m are each as defined above, are reacted with compounds of the formula

$$X(B)_n$$

where

X is as defined above, subject to the meaning of n,

n is 1, 2 or 3, and

B is a detachable Cl or F radical.

**9.** Aqueous dye preparations, especially dye solutions, containing

- 0.5 to 20% by weight of one or more dyes with the proviso that at least one thereof corresponds to the compound of Claim 1 or a salt thereof,
- 50 to 99.5% by weight of water,
- 0 to 30% by weight of one or more organic solvents,
- 0 to 30% by weight of viscosity and/or surface tension modifiers.

**10.** Printing inks containing at least one dye which corresponds to a compound according to Claim 1.

**11.** Use of the compounds of Claim 1 for dyeing and printing hydroxyl- and/or amido-containing substrates, especially for printing paper by the inkjet method.

**Revendications**

**1.** Composés de formule (I)

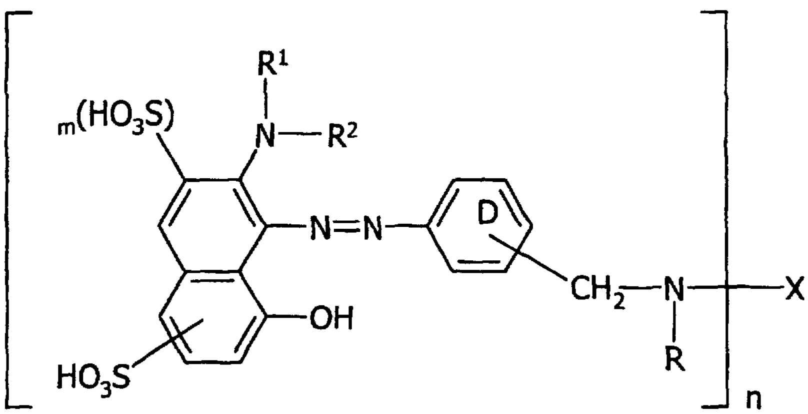

(I),

dans laquelle

| | |
|---|---|
| R | représente l'hydrogène ou un groupe alkyle en $C_1$-$C_6$ éventuellement substitué, |
| $R^1$ | représente l'hydrogène ou un groupe alkyle en $C_1$-$C_6$, |
| $R^2$ | représente l'hydrogène, un groupe alkyle en $C_1$-$C_6$ éventuellement substitué ou un groupe phényle éventuellement substitué, |
| le cycle D | peut, le cas échéant, porter un ou plusieurs substituants identiques ou différents choisis parmi les groupes alkyle en $C_1$-$C_6$, alcoxy en $C_1$-$C_6$, les halogènes, plus spécialement F, Cl et Br, et les groupes $SO_3H$, |
| m | est égal à 0 ou 1, |
| n | est égal à 1, 2 ou 3, et selon la valeur de n |
| X | représente un radical acyle mono-, di- ou tri-valant, un radical de pyrimidine ou de 1,3,5-triazine, qui n'est pas réactif avec les fibres. |

**2.** Composés selon la revendication 1, pour lesquels

R représente l'hydrogène, un groupe alkyle en $C_1$-$C_6$ ou hydroxyalkyle en $C_2$-$C_4$,

$R^1$ représente l'hydrogène ou un groupe alkyle en $C_1$-$C_6$,

$R^2$ représente l'hydrogène, un groupe alkyle en $C_1$-$C_6$, hydroxyalkyle en $C_2$-$C_4$, aminoalkyle en $C_2$-$C_4$, plus spécialement un groupe alkyle en $C_2$-$C_4$ portant un substituant $NR^3R^4$ pour lequel

$R^3$ et $R^4$ représentent chacun, indépendamment l'un de l'autre, l'hydrogène, un groupe alkyle en $C_1$-$C_4$ ou hydroxyalkyle en $C_2$-$C_4$, ou bien un groupe phényle portant éventuellement des substituants alkyle en $C_1$-$C_4$, alcoxy en $C_1$-$C_4$, COOH, $SO_3H$, F, Cl et/ou Br, et

Le cycle D porte éventuellement un ou plusieurs substituants identiques ou différents choisis parmi les groupes alkyle en $C_1$-$C_4$, alcoxy en $C_1$-$C_4$, F, Cl et Br.

**3.** Composés selon la revendication 1, **caractérisés en ce que**

X, lorsque n = 1, représente un groupe alcanoyle en $C_2$-$C_6$ portant éventuellement un substituant COOH, un groupe aroyle en $C_6$-$C_{10}$ portant éventuellement des substituants alkyle en $C_1$-$C_4$, alcoxy en $C_1$-$C_4$, $SO_3H$, COOH, F, Cl et/ou Br, ou un groupe de formule (II), (IIIa) ou (IIIb)

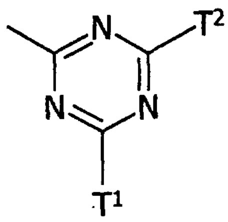
(II),

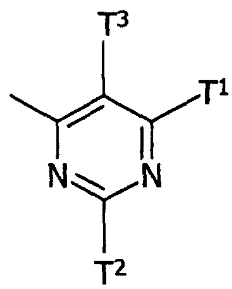
(IIIa),

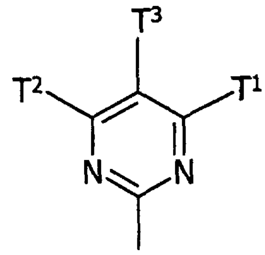
(IIIb),

dans lesquelles

$T^1$ et $T^2$ représentent chacun, indépendamment l'un de l'autre, un groupe $OR^5$, $NR^6R^7$ ou $SR^8$,

dans lesquels

$R^5$ et $R^8$ représentent chacun, indépendamment l'un de l'autre, l'hydrogène, un groupe alkyle en $C_1$-$C_6$, un groupe alkyle en $C_2$-$C_6$ portant des substituants sulfo, carboxy et/ou di(alkyle en $C_1$-$C_3$)amino, ou un groupe phényle portant éventuellement des substituants alkyle en $C_1$-$C_4$, alcoxy en $C_1$-$C_4$, sulfo, F, Br et/ou Cl,

$R^6$ représente l'hydrogène, un groupe alkyle en $C_1$-$C_6$ ou un groupe alkyle en $C_2$-$C_6$ portant éventuellement des substituants sulfo, hydroxy, alcoxy en $C_1$-$C_3$, amino ou mono- ou di-(alkyle en $C_1$-$C_3$)amino,

$R^7$ a, indépendamment de $R^6$, les mêmes significations que $R^6$ ou bien représente un groupe phényle ou naphtyle portant éventuellement des substituants alkyle en $C_1$-$C_4$, alcoxy en $C_1$-$C_4$, carboxy, sulfo, F, Cl et/ou Br, ou bien

$R^6$ et $R^7$ forment ensemble et avec l'atome d'azote auquel ils sont reliés un radical de morpholine ou un radical de pipérazine portant éventuellement des substituants alkyle en $C_1$-$C_4$, hydroxyalkyle en $C_2$-$C_4$ ou aminoalkyle en $C_2$-$C_4$ et

$T^3$ représente l'hydrogène, F, Cl, un groupe CN, $CCl_3$, $CCl_2F$ ou COOH,

X, lorsque n = 2 représente -CO-, $-CO-(CH_2)_{1 \text{ à } 4}-CO-$, $-COC_2H_2-CO-$, un groupe diaroyle en $C_6$-$C_{10}$ portant éventuellement des substituants $CH_3$, $OCH_3$, F, Cl, Br et/ou COOH, ou un groupe répondant à l'une des formules (IVa), (IVb) ou (V)

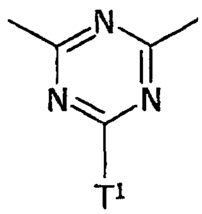

(IVa),

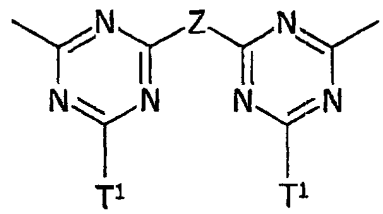

(IVb),

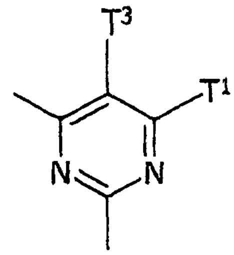

(V),

dans lesquelles $T^1$ et $T^3$ ont les significations indiquées ci-dessus et

Z représente un groupe de formule

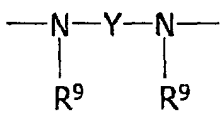

ou

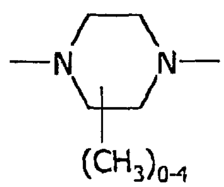

Y représente un groupe alkylène en $C_2$-$C_6$ ou un groupe arylène éventuellement substitué par des groupes carboxy ou sulfato,

$R^9$ représente l'hydrogène, un groupe alkyle en $C_1$-$C_6$ ou hydroxyalkyle en $C_2$-$C_4$,

X, lorsque n = 3, représente un groupe de formule (VI) ou (VII)

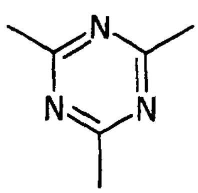

(VI)

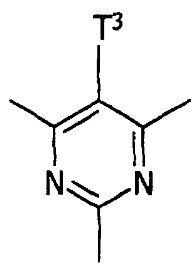

(VIII)

dans lesquelles $T^3$ a les significations indiquées ci-dessus.

**4.** Composés selon la revendication 1, **caractérisés en ce qu'**il répondent à la formule (VIII)

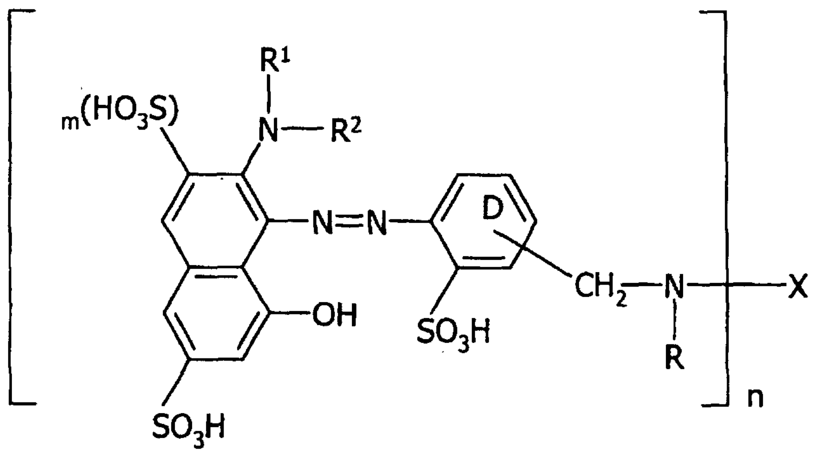

(VIII),

dans laquelle
R, $R^1$, $R^2$, D, X et m ont les significations indiquées ci-dessus,
n est égal à 1 ou 2

**5.** Composés selon la revendication 1, **caractérisés en ce que**

$R^1$ représente l'hydrogène, $CH_3$, $C_2H_5$ ou $C_3H_7$,
$R^2$ représente de préférence l'hydrogène, $CH_3$, $C_2H_5$, $C_3H_7$, $C_2H_4OH$, $C_2H_4SO_3H$, $C_3H_6OH$, $C_3H_6SO_3H$, $C_2H_4COOH$, $C_3H_6COOH$, $C_6H_5$, $C_6H_4SO_3H$, $C_6H_4COOH$ ou $C_2H_4N(CH_3)_2$,
R représente de préférence l'hydrogène, $CH_3$, $C_2H_5$, $C_2H_4OH$,
$R^3$ et $R^4$ représentent chacun, indépendamment l'un de l'autre, de préférence l'hydrogène, $CH_3$, $C_2H_4OH$,
$R^5$ dérive de préférence des composés $HOR^5$ suivants :

éthylèneglycol ou acide iséthionique (acide 2-hydroxyéthane sulfonique)

$R^6$ représente de préférence l'hydrogène, $CH_3$, $C_2H_5$, $C_3H_7$, $C_2H_4OH$, $C_2H_4OCH_3$ ou $C_3H_6OH$,
$R^7$ représente de préférence $C_2H_4OH$, $C_3H_6OH$, $C_2H_4SO_3H$, $C_2H_4COOH$, $C_3H_6SO_3H$, $C_3H_6COOH$, $C_6H_5$, $C_6H_4SO_3H$, $C_6H_4COOH$, ou bien
$R^6$ et $R^7$ forment ensemble et avec l'atome d'azote auquel ils sont reliés de préférence un groupe de formule

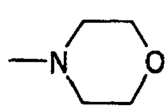 , 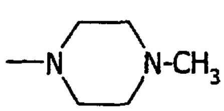 , 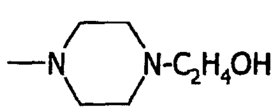 ou 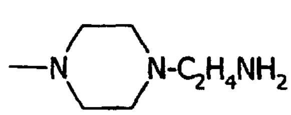

$R^8$ dérive de préférence des thiols $HSR^8$ suivants : 2-mercaptoéthanol ou acide 3-mercapto-1-propane sulfonique,
$R^9$ représente de préférence l'hydrogène, $CH_3$, $C_2H_4OH$.

**6.** Composés selon la revendication 1, **caractérisés en ce qu'**ils répondent à la formule (IX)

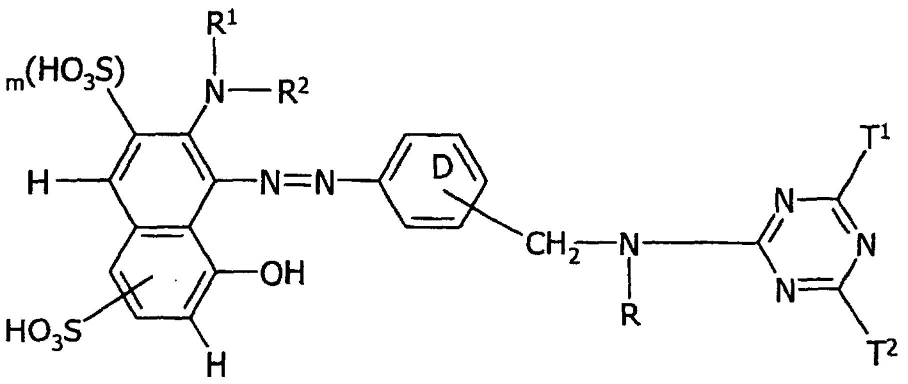

(IX),

dans laquelle les symboles ont les significations indiquées ci-dessus.

**7.** Composés selon la revendication 1, **caractérisés en ce qu'**ils répondent à la formule (X)

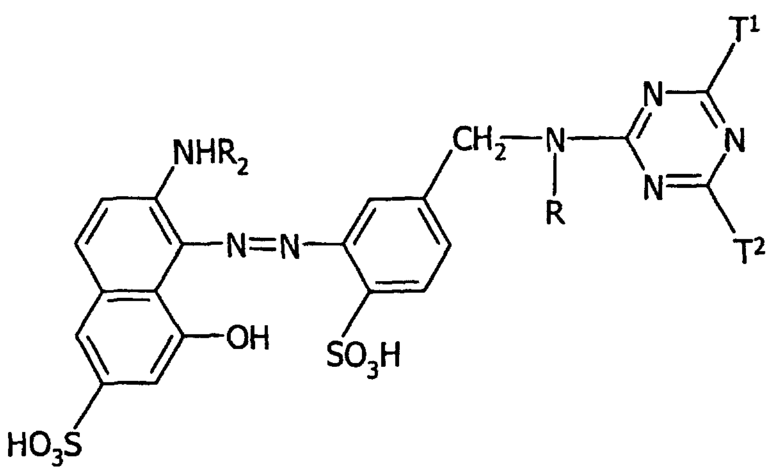

(X),

dans laquelle

| | |
|---|---|
| R | représente l'hydrogène, $CH_3$ ou $C_2H_5$, |
| $R^2$ | représente l'hydrogène, $CH_3$, $C_2H_5$, $C_3H_7$, $C_4H_9$ ou $C_6H_5$ et |
| $T^1$ et $T^2$ | ont les significations indiquées ci-dessus. |

**8.** Procédé pour la préparation des composés selon la revendication 1, **caractérisé en ce que** l'on fait réagir des composés de formule (XI)

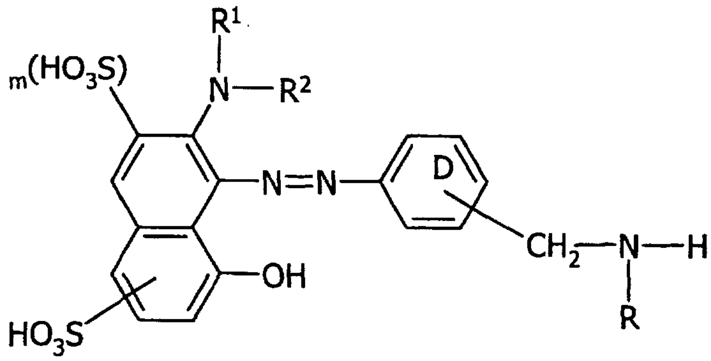

(XI),

dans laquelle

R, $R^1$, $R^2$, D et m ont les significations indiquées ci-dessus, avec des composés de formule

$$X(B)_n$$

dans laquelle

X, selon la valeur de n, a les significations indiquées ci-dessus,
n est égal à 1, 2 ou 3 et
B représente un atome de Cl ou de F éliminable.

**9.** Compositions tinctoriales aqueuses, en particulier solutions de colorants contenant

- 0,5 à 20 % en poids d'un ou plusieurs colorants dont l'un au moins est un composé selon la revendication 1, éventuellement à l'état de sel,
- 50 à 99,5 % en poids d'eau,
- 0 à 30 % en poids d'un ou plusieurs solvants organiques,
- 0 à 30 % en poids d'additifs agissant sur la viscosité et/ou sur la tension superficielle

**10.** Encres d'impression contenant au moins un colorant consistant en un composé selon la revendication 1.

**11.** Utilisation des composés de la revendication 1 pour la teinture et l'impression de supports à groupes hydroxy et/ou amides, et en particulier pour l'impression du papier par la technique du jet d'encre.